# EUROPEAN PATENT SPECIFICATION

(11) **EP 0 444 899 B1**
(45) Date of publication and mention of the grant of the patent: **05.02.1997**
(21) Application number: 91301587.1
(22) Date of filing: 27.02.1991
(51) Int. Cl.: A61K 31/06, A61K 31/12, A61K 31/165, A61K 31/095, A61K 31/44, A61K 31/47, A61K 31/495, A61K 31/445, C07C 205/45, C07C 49/258, C07C 49/583

(54) **Catechol derivatives, their physiologically acceptable salts, esters and their use in the treatment of tissue damage induced by lipid peroxidation**
Katecholderivate, deren physiologisch unbedenkliche Salze, Ester und deren Verwendung bei der Behandlung von durch Lipidperoxidation verursachten Gewebeschäden
Dérivés du catéchol, ses sels, esters physiologiquement acceptables et leur utilisation pour le traitement du dammage du tissu induit par la peroxidation de la graisse

(30) Priority: 27.02.1990 GB 9004348
(43) Date of publication of application: 04.09.1991
(73) Proprietor: ORION-YHTYMÄ OY, 02101 Espoo (FI)
(72) Inventor: Korkolainen, Tapio Juhani, SF-00250 Helsinki (FI); Nissinen, Erkki Aarne Olavi, SF-02180 Espoo (FI); Backstrom, Reijo Johannes, SF-00360 Helsinki (FI); Pippuri, Aino Kyllikki, SF-02260 Espoo (FI)
(74) Representative: Sexton, Jane Helen

(56) References cited:
- EP-A- 0 323 162
- EP-A- 0 326 379
- FR-A- 2 607 493
- US-A- 4 618 627
- FASEB J., vol. 1, 1987, pages 358-364; B. HALLIWELL: "Oxidants and human disease: some new concepts"
- PATENT ABSTRACTS OF JAPAN, vol. 11, no. 192 (C-429)[2639], 19th June 1987; & JP-A-62 012 757
- PATENT ABSTRACTS OF JAPAN, vol. 13, no. 523 (C-657)[3871], 21st November 1989; & JP-A-1 213 276
- DIG. DIS. SCI., vol. 35, no. 8, August 1990, page 1036; T. METSA-KETELA et al.: "Radical-trapping antioxidant properties of nitecapone"
- FASEB J., vol. 1, 1987, pp. 358-364

## Description

The present invention relates to catechol derivatives and their physiologically acceptable salts and esters, which are useful as medicinal antioxidants.

French Patent specification 2607493 discloses substituted catechol derivatives which are useful in the treatment of Parkinson's disease, depression, cardiac failure and hypertension. This utility is associated with the ability of the derivatives described to inhibit the enzyme catechoi-0-methyi transferase (COMT).

British Patent specification 2220569A discloses that COMT-inhibitors, including especially substituted catechols, are useful in the treatment of breast cancer.

United States patent No. 4618627 discloses substituted catechol derivatives stated to be useful for the prophylaxis and treatment of various allergic diseases, ischemic heart diseases and inflammations caused by slow reacting substance of anaphylaxis (SRS-A), the production and release of which is inhibited by the disclosed compounds.

Medicinal antioxidants are compounds that may be used for the prevention or treatment of tissue damage induced by lipid peroxidation. Cellular damage by oxygen derived radicals especially those associated with lipid peroxidation are generally believed to be a significant factor in heart disease, rheumatoid arthritis, cancer, certain inflammatory diseases, rejection reactions in organ transplants, ischemia and even in the aging process (Halliwell, B., FASEB J. 1:358-364, 1987). During lipid peroxidation free radicals interact with polyunsaturated fatty acids to form lipid peroxyl radicals, which produce lipid hydroperoxides and further lipid peroxyl radicals. This peroxidative cascade may eventually consume an essential part of the membrane lipid, which may lead to changes in membrane permeability and ultimately in cell death. The peroxidative degradation of lipids also leads to the formation of potentially toxic products such as malondialdehyde (Comporti,M., Lab Invest. 53:599-623, 1985).

It has now been found that some catechol derivatives are surprisingly effective as medicinal antioxidants which are effective in the prevention or treatment of tissue damage in a condition which is rheumatoid arthritis, a rejection reaction in an organ transplant, ischemia or ageing, the tissue damage being induced by lipid peroxidation. Thus the present invention provides the use of a catechol derivative which is a compound of formula I: 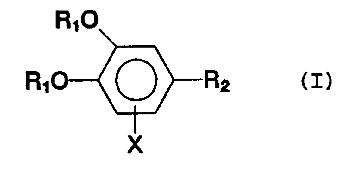 in which R₁ is hydrogen, X is nitro, cyano, trifluoromethyl, formyl or carboxyl and R₂ is
a) halogen, substituted lower alkyl, lower alkoxy, an aryl or heterocyclic ring, or a nitro, cyano, formyl or carboxyl group or
b) a group selected from 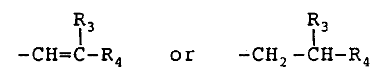 in which R₃ is hydrogen, lower alkyl, cyano, carboxyl, cycloalkyl carbonyl or lower acyl and R₄ is hydrogen, cyano, carboxyl, formyl, lower alkoxy, lower alkoxycarbonyl, lower alkoxycarbonyl lower alkyl, lower carboxy alkenyl, carboxycarbonyl, nitro, lower acyl, lower hydroxyalkyl, lower carboxyalkyl, or one of following optionally substituted groups; carbamoyl, aryl, heterocyclic ring, cycloalkyl, cycloalkyl carbonyl, aroyl, or COZ, in which Z is an optionally substituted heterocyclic ring or c) 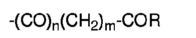 in which n is 0-1, m is 0-7 and R is lower alkyl, hydroxy, lower carboxyalkyl, optionally substituted lower alkenyl, optionally substituted heterocyclic ring or aryl, lower alkoxy or optionally substituted amino 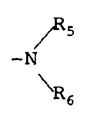 in which R₅ and R₆ independently comprise hydrogen or one of the following optionally substituted groups; lower alkyl, lower alkenyl, lower alkynyl, cycloalkyl, aralkyl or taken together with the nitrogen to which they are attached form an optionally substituted heterocyclic ring, or a physiologically acceptable salt or ester thereof in the manufacture of a medicament for use in the prevention or treatment of tissue damage in a condition which is rheumatoid arthritis, a rejection reaction in an organ transplant, ischemia or ageing, the tissue damage being induced by lipid peroxidation.

Most of the above compounds are known and have been described in general in e.g. GB-A-2200109, EP-A-0323162 and in EP-A-0237929. They have been shown to be effective medicaments for treating Parkinsonism (cf. French patent specification 2607493 mentioned above) as well as in the treatment and prophylaxis of ulcers and lesions in the gastrointestinal tract.

The present invention also provides, as new compounds, the catechol derivatives of formula I, in which R₁ is as defined above, X is nitro, cyano, trifluoromethyl, formyl, or carbonyl and R₂ is 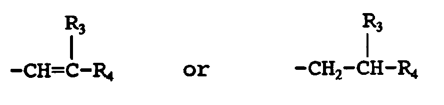 in which R₃ is lower acyl or cyclopropyl carbonyl and R₄ is optionally substituted aryl or cyclopropyl carbonyl and physiologically acceptable salts and esters thereof.

The present invention further provides a catechol derivative which is 3-(3,4-dihydroxy-5-nitrobenzyl)-2,4-pentanedione or 4-(3,4-dihydroxy-5-nitrobenzyl)-3,5-heptanedione or a physiologically acceptable salt or ester thereof.

Among preferred compounds of formula I for use in the invention are those in which X is nitro or cyano, especially in the 5 position. In these compounds R₂ is especially nitro, cyano, substituted lower alkyl or a group 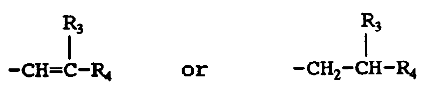 in which R₃ is carboxyl, cycloalkyl carbonyl or lower acyl and R₄ is carboxyl, lower acyl, formyl, lower alkoxycarbonyl, carboxycarbonyl, one of optionally substituted carbamoyl, cycloalkylcarbonyl, aroyl or COZ, in which Z is an optionally substituted heterocyclic ring. Also preferred are compounds in which R₂ is 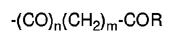 in which n is 0 and m is 0 and R is optionally substituted amino or n is 0, m is 1 to 4 and R is lower alkyl, hydroxy, lower carboxyalkyl, optionally substituted lower alkenyl, optionally substituted heterocyclic ring or aryl, lower alkoxy or optionally substituted amino.

Among these compounds may be mentioned e.g.
3-nitro-5-[2-(4-pyridyl)vinyl]catechol,
3-nitro-5-[2-(4-quinolyl)vinyl]catechol,
3,4-dihydroxy-5-nitrocinnamic acid,
3,4-dihydroxy-5-nitro-ω,ω-dicyanostyrene,
4-(3,4-dihydroxy-5-nitrophenyl)-3-methylbut-3-en-2-one,
3-(3,4-dihydroxy-5-nitrophenyl)methylene-2,4-pentanedione,
3-(3,4-dihydroxy-5-nitrophenyl)-1-phenylprop-2-en-1-one,
3-(3,4-dihydroxy-5-nitrophenyl)-1-(4-methoxyphenyl)-prop-2 -en-1-one,
3-(3,4-dihydroxy-5-nitrophenyl)-1-(3,4-dimethoxyphenyl)-prop-2-en-1-one,
3-(3,4-dihydroxy-5-nitrophenyl)-1-(3,4,5-trimethoxyphenyl)-prop-2-en-1-one,
3-(3,4-dihydroxy-5-nitrophenyl)-1-(2-hydroxyphenyl)-prop-2-en-1-one,
3-(3,4-diacetoxy-5-nitrophenyl)-1 -phenylrop-2-en-1-one,
3-(3,4-dibenzoyloxy-5-nitrophenyl)-1-phenylprop-2-en-1-one,
3-(3-pivaloyloxy-4-hydroxy-5-nitrophenyl)-1-phenyl-prop-2-en-1-one,
4-(3,4-dihydroxy-5-nitrophenyl)-3-methylbut-3-en-2-ol,
3',4'-dihydroxy-5'-nitroacetophenone,
5-(3,4-dihydroxy-5-nitrophenyl)pentanoic acid,
1-benzyl-4-[5-(3,4-dihydroxy-5-nitrophenyl)pentanoyl]piperazine hydrochloride,
N-isopropyl-5-(3,4-dihydroxy-5-nitrophenyl)pentanoic amide,
N-methyl-N-propargyl-5-(3,4-dihydroxy-5-nitrophenyl)pentanoic amide,
N-(1-adamantyl)-5-(3,4-dihydroxy-5-nitrophenyl)pentanoic amide,
3-(3,4-dihydroxy-5-nitrophenyl)-1-(4-methylphenyl)-prop-2 -en-1-one,
N-(1-adamantyl)-3,4-dihydroxy-5-nitrobenzamide,
4-cyclohexylcarbonyl-1-(3,4-dihydroxy-5-nitrobenzoyl)-piperidine,
N-benzyl-3,4₋dihydroxy-5-nitrobenzamide,
N-(1-adamantyl)-3,4-dihydroxy-5-cyanobenzamide,
1-butyl-3,4-dihydroxy-5-cyanobenzoate,
3-(3-ethoxycarbonylmethylcarbamoyloxy-4-hydroxy-5-nitrophenyl)-1-phenylprop-2-en-1-one,
3-(3,4-dihydroxy-5-nitrophenyl)-1-(2-carboxyphenyl)-prop-2 -en-1 -one,
3-(3,4-dihydroxy-5-nitrophenyl)-1-(4-nitrophenyl)-prop-2 -en-1-one,
ethyl 2-cyano-3-(3,4-dihydroxy-5-nitrophenyl)acrylate,
methyl 3-(3,4-dihydroxy-5-nitrophenyl)methylene-4-ketopentanoate,
3,4-dihydroxy-5-nitrobenzylmalonitrile,
ethyl 3,4-dihydroxy-5-nitrobenzylcyanoacetate,
3-(3,4-dihydroxy-5-trifluoromethylphenyl)-prop-2-en-1-one,
3,4-dihydroxy-5-cyanobenzaldehyde,
3-(3,4-dihydroxy-5-trifluoromethylbenzaldehyde,
2-cyano-3-(3,4-dihydroxy-5-nitrophenyl)acrylamide,
N, N-dimethyl-2-cyano-3-(3,4-dihydroxy-5-nitrophenyl)-acrylamide,
N,N-diethyl-2-cyano-3-(3,4-dihydroxy-5-nitrophenyl)acrylamide,
N-isopropyl-2-cyano-3-(3,4-dihydroxy-5-nitrophenyl)acrylamide,
N'-methyl-N"-[2-cyano-3-(3,4-dihydroxy-5-nitrophenyl)-acryl] piperazine
3-(3,4-dihydroxy-5-trifluoromethylphenyl)methylene-2,4-pentanedione,
3,4-dihydroxy-5-nitrobenzylalcohol,
3,4-dihydroxy-5-nitrobenzyl-2-methoxyethyl ether,
3,4-dihydroxy-5-nitrobenzylthioacetic acid,
2-(3,4-dihydroxy-5-nitrobenzyl)pyrrole,
2-cyano-3-(3,4-dihydroxy-5-nitrophenyl)propanol,
3,5-dinitrocatechol,
3,4-dihydroxy-5-nitrobenzaldehyde,
3,4-dihydroxy-5-nitrobenzonitrile,
4-chloro-6-nitrocatechol,
4,5-dihydroxyisophtalaldehyde,
3,4-dihydroxy-5-cyanobenzoic acid,
3,5-dicyanocatechol,
N-(3-hydroxypropyl)-3,4-dihydroxy-5-nitrobenzamide, neopentyl 2-cyano-3-(3,4-dihydroxy-5-nitrophenyl)acrylate,
N-(3-hydroxypropyl)-2-cyano-3-(3,4-dihydroxy-5-nitrophenyl)acrylamide and
5-(3,4-dihydroxy-6-nitrophenyl)pentanoic acid,
N-(1-adamantyl)-3,4-dihydroxy-6-nitrobenzamide,
N-(4-morpholine ethyl)-3,4-dihydroxy-6-nitrobenzamide-hydromesvlate.
1-(3,4-dihydroxy-6-nitrobenzoyl)-4-cyclohexylcarbonylpiperidine and
1-(3,4-dihydroxy-6-nitrobenzoyl)-4-(1-piperidyl)piperidine hydromesylate. A general method of preparation of the
above compounds has been described in GB-A-2200109.

Also preferred compounds are N-(1-adamantyl)-3,4-dihydroxy -5-cyanobenzamide,
3,4-dihydroxy-5-nitrophenylmethanethiol,
4-[(3,4-dihydroxy-5-cyanophenyl)methylene]-3,5-heptanedione,
5-(3,4-dihydroxy-5-nitrophenyl)-2-methyl-4-phenyl-4-penten-3-one,
4-[(3,4-dihydroxy-5-cyanophenyl)methylene]-3,5-pentanedione,
1-(3,4-dihydroxy-5-nitrophenyl)-2-methylene-1-propanone,
3-(3,4-dihydroxy-5-nitrobenzyl)-2,4-pentanedione,
4-(3,4-dihydroxy-5-nitrophenyi)methyiene-3,5-heptanedione and
4-(3,4-dihydroxy-5-nitrobenzyl)-3,5-heptanedione, the preparation of which is described below.

The term "alkyl" as employed herein by itself or as part of another group refers to both straight and branched carbon chain groups. The term "lower alkyl" refers to an alkyl group of up to 7 carbon atoms, more preferably 1 to 4 carbon atoms, most preferably 1 to 2 carbon atoms.

The terms "alkenyl" and "alkynyl" as employed herein refer to an alkyl as defined above having at least one carbon to carbon double group and carbon to carbon triple bond, respectively

The term "acyl" as employed herein refers to an alkylcarbonyl or alkenylcarbonyl group, the alkyl and alkenyl groups being defined above. The term "aroyl" refers to an arylcarbonyl group, the aryl group being a monocyclic or bicyclic group containing 6 to 10 carbon atoms in the ring portion.

The term "alkoxy" as employed herein refers to an alkyl residue as defined above linked to an oxygen atom.

The term "cycloalkyl" as employed herein refers to saturated cyclic hydrocarbon groups having 3 to 10, preferably 5 to 7 carbon atoms.

The term "aralkyl" as employed herein refers to alkyl groups as defined above having an aryl substituent.

The term "heterocyclic" ring as employed herein refers to a monocyclic or bicyclic ring having 1 to 3, preferably 1 to 2 heteroatoms N and/or O and /or S. The ring may be aromatic or nonaromatic. The term "heteroaromatic" refers to an aromatic heterocyclic ring. Specific examples are morpholinyl, piperidyl, piperazinyl, pyridyl, pyrrolyl and quinolyl.

The "optional substituent" as employed herein refers to halogen, trifluoromethyl, nitro, cyano, hydroxy, carboxy, lower alkyl, lower hydroxyalkyl, thiol, lower alkyl thio, lower carboxyalkyl thio, lower alkoxy, lower alkenyl, lower alkynyl, aryl, heteroaryl, lower alkyl aryl, halogen aryl, cycloalkyl, cycloalkyl carbonyl, lower alkylcycloalkyl, lower alkylamino, lower alkanoylamino, arylcarbonylamino, lower alkoxy lower alkoxy, aryl lower alkyl and alkoxy carbonyl.

For example, when R₂ is lower alkyl the substituents are preferably hydroxy, halogen, cyano, thiol, lower alkyl thio, lower alkoxy lower alkoxy, lower alkoxy, lower carboxyalkyl thio and heteroaryl.

When R₄ is optionally substituted carbamoyl the substituents are preferably for example lower alkyl and lower hydroxyalkyl. The optional substituents of aryl and aroyl are preferably halogen, trifluoromethyl, nitro, cyano, hydroxy, carboxy, lower alkyl, lower hydroxyalkyl, lower alkoxy, lower alkyl amino and those of the heterocyclic ring are preferably the same and further lower alkyl, aryl lower alkyl and cycloalkylcarbonyl. The optional substituents of cycloalkyl are preferably lower alkyl and halogen.

When R is optionally substituted alkenyl the substituents are preferably halogen, carboxy, alkoxy carbonyl and lower alkoxy. The preferred optional substituents for R being a heterocyclic ring and aryl are the same as given above.

When R₅ and/or R₆ are optionally substituted lower alkyl, lower alkenyl, cycloalkyl, heterocyclic ring or aralkyl the optional substituents are preferably the same as given above and those of lower alkynyl the same as for lower alkenyl.

GB-A-2200109, EP-A-0323162 andEP-A-0237929 disclose a general method whereby the compounds according to the invention can be prepared. For example the compounds of formula I, in which R₂ is 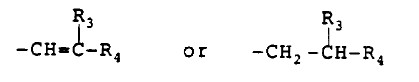 in which R₃ and R₄ are as defined above, may be prepared by condensing a compound of formula II 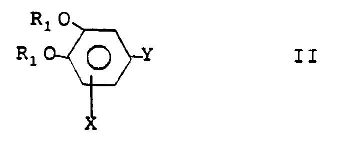 in which R₁ and X are as defined above, and Y is -CHO or -CH₂Q, in which Q is hydroxy, halogen or an alkyl or aryl sulfonate group, in the presence of acidic or basic catalyst with a compound of formula III 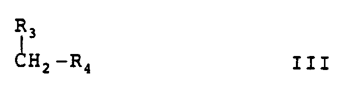 in which R₃ and R₄ are as defined above, giving the compounds of formula la and Ib, respectively, 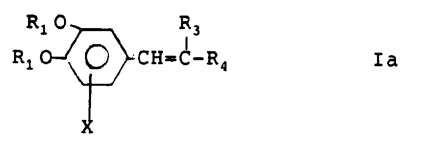 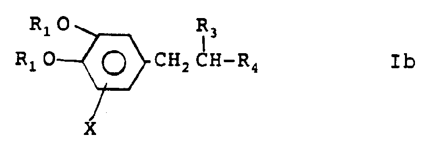 in which R₁, X, R₃ and R₄ are as defined above.

Alternatively, the compound of formula Ib may be prepared by reducing the double bond of the compound of formula la to a single bond, using the known reduction agents, for example sodium borohydride. It is also possible to prepare the compound la from the compound Ib by halogenating the compound of formula Ib to give the compound of formula IV 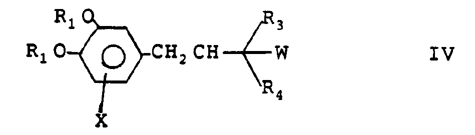 in which R₁, X, R₃ and R₄ are as defined above and W is halogen and dehydrohalogenating compound IV to produce a compound of formula la.

Methods for the preparation of esters of the compounds according to the invention have been described in the above mentioned patent applications. Preferred esters are lower acyl, lower alkyl carbamoyl or aroyl derivatives which will hydrolyse readily under physiological conditions.

Salts of these compounds, when applicable, may be prepared by known methods. All physiologically acceptable salts are useful as active medicaments, however, sodium, potassium, ammonium, calcium and magnesium salts and salts with hydrochloric, hydrobromic, phosphoric and sulfuric acids and with organic acids like oxalic, fumaric, tartaric, malonic, acetic and citric acids etc. are preferred.

The effective dose of the compound varies considerably depending on whether the compounds are given for prophylaxis or for treatment, the severity of the condition to be treated, and the route of administration. The effective dose for human beings is likely to be from about 1 to 1000 mg per day.

The compounds used in this invention are formulated into dosage forms using the principles which are known to the man having average skill in the art. The compounds according to this invention are given to a patient as such or in combination with suitable pharmaceutical material in the form of tablets, dragees, capsules, suppositories, emulsions, suspensions or solutions whereby the contents of the active compound is in the formulation from 1 to 100 weight %.

Choosing the auxiliary ingredients for the formulation is routine for those of ordinary skill in the art. It is evident that suitable solvents, gel forming ingredients, dispersion forming ingredients, colors etc. are used in a normal way

The compositions may be administered enterally or parenterally.

### Radical trapping capacity of compounds

The tested compounds were subjected to controlled peroxidation by peroxyl radicals originating from the thermal decomposition of 2,2'-azobis-(2-amidinopropane)^{*}2HCI at 37°C. The rate of radical formation was followed by luminol enhanced chemiluminescence (CL). From the duration of CL and from the fact that the phenolic antioxidant vitamin E analogue TROLOX^{R} traps two radicals (Barclay,L. et al., J. Am. Chem. Soc. 106: 2479-2481, 1984) the stochiometric factors were calculated. The results are presented in Table 1.

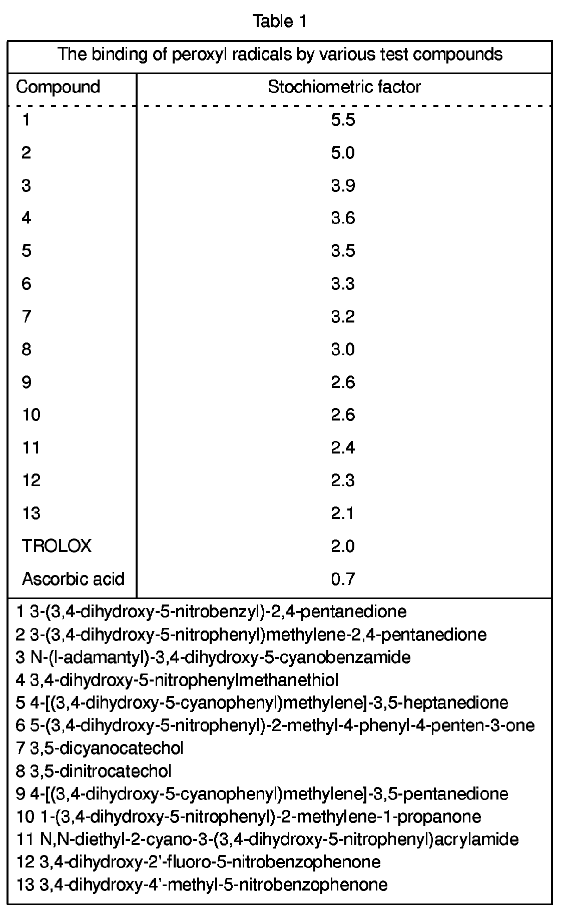

The total peroxyl radical trapping antioxidant parameter (TRAP) for the test compound 1 was studied in vivo by dosing 1 mg/kg or 10 mg/kg i.v. to rats. Plasma samples were taken at 0, 5, 15, 30 and 60 min after dosing. The plasma TRAP was calculated as µM of trapped radicals.

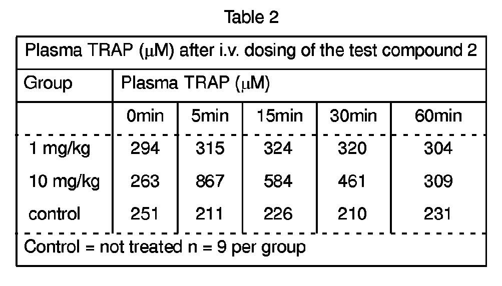

The following examples illustrate the production of compounds of use according to the invention.

### Example 1

### 4-(3,4-Dihydroxy-5-nitrophenyl)-3-phenyl-3-buten-2-one

A solution containing 2.68 g of 3-phenyl-2-propanone and 3.66 g of 3,4-dihydroxy-5-nitrobenzaldehyde in 80 ml of 2-propanol was saturated with hydrogen chloride gas at 20°C. The mixture was stirred for 4 h at room temperature, filtered and washed with 2-propanol. The product was recrystallized from methanol. Yield 1.34 g, mp 170-176°C.

### Example 2

### 4-(3,4-Dihydroxy-5-nitrophenyl)-3-(4-methylphenyl)-3-buten-2-one

The procedure described in Example 1 was repeated by using 0.81 g of 3-(4-methylphenyl)-2-propanone and 0.92 g of 3,4-dihydroxy-5-nitrobenzaldehyde. Yield 0.82 g, mp 189-194°C.

### Example 3

### 5-(3,4-Dihydroxy-5-nitrophenyl)-2-methyl-4-phenyl-4-penten-3-one

The procedure described in Example 1 was repeated by using 2.96 g of 2-methyl-4-phenyl-3-butanone and 2.4 g of 3,4-dihydroxy-5-nitrobenzaldehyde. Yield 0.52 g, mp 151-156°C.

### Example 4

### 3-(3,4-Dihydroxy-5-nitrobenzyl)-2,4-pentanedione

A mixture containing 6.0 g of 3,4-dihydroxy-5-nitrobenzylalcohol and 1.0 ml of trifluoroacetic acid in 70 ml of 2,4-pentanedione was heated for 20 h at 100°C. The solvents were evaporated in vacuo and the residue was crystallized from 2-propanol. Yield 3.0 g, mp 118-128°C.

### Example 5

### 4-[(3,4-Dihydroxy-5-cyanophenyl)methylene]-3,5-heptanedione

A mixture 1.6 g of 3,4-dihydroxy-5-cyanobenzalde, 2.5 g of 3,5-heptanedione, 0.2 ml of piperidine and 0.6 ml of acetic acid in 70 ml of toluene was refluxed for 2 h with Dean-Stark separator. The solvent was evaporated in vacuo and the residue was crystallized from acetone-cyclohexane. Yield 0.33 g, mp 165-173°C.

### Example 6

### 1,3-Dicyclopropyl-2-[(3,4-dihydroxy-5-nitrophenyl)methylene]-1,3-propanedione

A mixture containing 2.75 g of 3,4-dihydroxy-5-nitrobenzaldehyde, 3.8 g of 1,3-dicyclopropyl-1,3-propanedione, 0.2 ml of piperidine and 0.4 ml of acetic acid was refluxed for 1 h with a Dean-Stark separator. After cooling the product was filtered and recrystallized from acetic acid. Yield 2.85 g, mp 159-162°C.

### Example 7

### 3,4-Dihydroxy-5-nitrophenyl methanethiol

### a) 3,4-Dihydroxy-5-nitrobenzyl bromide

12.2 g of 3,4-dihydroxy-5-nitrobenzyl alcohol was dissolved in 100 ml of 47 % hydrobromic acid at 50°C. After cooling the product was filtered and washed with 47 % hydrobromic acid. The crude product was dissolved in dichloromethane and dried over Na₂S0₄. The mixture was filtered and the solvent was evaporated in vacuo. Yield 7.1 g.

### b) 3,4-Dihydroxy-5-nitrophenylmethanethiol

2.5 g of the above product was gradually added to a solution containing 3.0 g of sodiumhydrogen sulfide in 30 ml of N-methylpyrrolidone and 20 ml of water. The solution was stirred for 45 min at 20°C. 100 ml 1 N hydrochloric acid was then added and the solution was extracted twice with 100 ml of ether. The ether extract was washed with conc. Na₂S0₄- solution, dried over Na₂S0₄. After filtering the solvent was evaporated in vacuo and the residue was crystallized from water. Yield 0.35 g, mp 179-185°C.

### Example 8

### N-(1-Adamantyl)-3,4-di hydroxy-5-cyanobenzamide

### a) N-(1-Adamantyl)-3,4-diacetoxy-5-cyanobenzamide

A solution containing 0.8 g of 3,4-diacetoxy-5-cyanobenzoic acid and 0.32 ml of thionyl chloride and a catalytic amount of N,N-dimethylformamide in 10 ml of toluene was heated for 1 h at 80°C. The solvent was evaporated in vacuo and the residue was dissolved in 5 ml of dichloromethane and added to a mixture containing 0.56 g of 1-aminoadaman- tane hydrochloride and 0.94 ml of triethylamine in 10 ml of dichloromethane and stirred for 15 min at 0°C and then 15 min at 20°C. Water was added to the reaction mixture and dichloromethane phase was separated. The solvent was evaporated in vacuo yielding yellow viscous oil 1.0 g.

### b) N-(1-Adamantyl)-3,4-dihydroxy-5-cyanobenzamide

A solution containing 1.2 g of the above product and a catalytic amount of sulfuric acid in 10 ml of methanol was refluxed for 3 h. 20 ml of water was added and on cooling 0.75 g of the desired product was crystallized, m.p. 253-255°C.

### Example 9

### 4-[(3,4-Dihydroxy-5-cyanophenyl)methylene]-3,5-pentanedione

A mixture 1.6 g of 3,4-dihydroxy-5-cyanobenzaidehyde, 2.0 g of 3,5-pentanedione, 0.2 ml of piperidine and 0.6 ml of acetic acid in 70 ml of toluene was refluxed for 2 h with Dean-Stark separator. The solvent was evaporated in vacuo and the residue was crystallized from acetone-cyclohexane. mp 228-232 °C.

### Example 10

### 1-(3,4-Dihydroxy-5-nitrophenyl)-2-methylene-1-propanone

### a) 3',4'-Dimethoxy-2-bromoisobutyrophenone

A mixture of veratrole (1,3-dimethoxybenzene) (13.8 g), 2-bromoisobutyryl bromide (23.0 g) and aluminium chloride (14.0 g) was heated for 1 hour at 70-80°C. After cooling, ice and hydrochloric acid were added to the reaction mixture which was then extracted with ether. The extract was washed first with water and then with 1 N NaOH-solution, dried and the solvent evaporated in vacuo, yielding a pale yellow oil (12.3 g).

### b) 4'-Hydroxy-3'-methoxy-2-bromoisobutyrophenone

A solution of 3',4'-dimethoxy-2-bromoisobutyrophenone (11.5 g) and aluminium bromide (21.8 g) in toluene (220 ml) was refluxed for 15 min. After cooling the reaction mixture was poured into ice (300 g) and 1 N hydrochloric acid (200 ml).

The organic phase was separated and dried over Na₂S0₄. The solvent was evaporated in vacuo yielding a pale yellow oil (10.0 g).

### c) 4'-Hydroxy-3'-methoxy-5'-nitro-2-bromoisobutyrophenone

To a solution of 4'-hydroxy-3'-methoxy-2-bromoisobutyrophenone (10.0 g) in dichloromethane (80 ml) was gradually added 2 M HN0₃-dichloromethane (19 ml). The mixture was stirred for 2 hours at 10°C after which water (100 ml) was added. The organic phase was separated, dried over Na₂S0₄ and evaporated to dryness. Ether (20 ml) was added. The resulting crystals were filtered and washed with ether. Yield 3.3 g.

### d) 3',4'-Dihydroxy-5'nitro-2-bromoisobutyrophenone

To a solution of 4'-dihydroxy-3'-methoxy-5'-nitro-2-bromoisobutyrophenone (3.0 g) in dichloromethane (60 ml) was gradually added 1 M BBr₃-dichloromethane (30 ml). The solution was stirred over night at room temperature. Water and hydrochloric acid were added to the reaction mixture. The organic phase was separated and evaporated to dryness. Yield 2.6 g.

### e) 1-(3,4-Dihydroxy-5-nitrophenyl)-2-methylene-1-propanone

A solution of 3',4'-dihydroxy-5'-nitro-2-bromoisobutyrophenone (2.6 g) and triethylamine (20 ml) in pyridine (20 ml) was heated for 3 hours at 100°C. Triethylamine and pyridine were evaporated in vacuo. Ether and 2N H₂SO₄ were added. The organic phase was separated and dried over Na₂S0₄. The ether solution was treated with charcoal for 2 hours, filtered and evaporated to dryness. The residue was crystallized from dichloromethane-petroleum ether (40-60°C). Yield 0.48 g, mp 98-102°C.

### Example 11

### 4-(3,4-Dihydroxy-5-nitrophenyl)methylene-3,5-heptanedione

To a solution of 3,4-dihydroxy-5-nitrobenzaidehyde (7.6 g) and 3,5-heptanedione (8.0 g) in 50 ml of 2-propanol was added thionyl cloride (3.5 ml) with stirring at 20°C. Ether (60 ml) was added and the solution was filtered and allowed to stand overnight. The resulting crystal were filtered. Yield 1.2 g, mp 133-135°C.

### Example 12

### 4-(3,4-Dihydroxy-5-nitrobenzyl)-3,5-heptanedione.

A mixture containing 0.2 g of 3,4-dihydroxy-5-nitrobenzyl alcohol and 0.03 ml of trifluoroacetic acid in 2.5 ml of 3,4-heptanedione was heated for 20 h at 100 °C. The solvent was evaporated in vacuo and the residue was recrystallized from 2-propanol. Yield 0.3 g, mp 126-131 °C.

### Example 13

### 3-(3,4-Dihydroxy-5-nitrobenzyl)-1-cyclopropyl-1,3-butanedlone

The procedure described in Example 4 was repeated by using 0.18 g of 3,4-dihydroxynitrobenzylalcohol and 2ml of 1-cyclopropyl-1,3-butanedione. The residue was crystallized from diethylether. Yield 52 mg, mp 98-104 °C.

## Claims

1. Use of a catechol derivative which is a compound according to the formula I 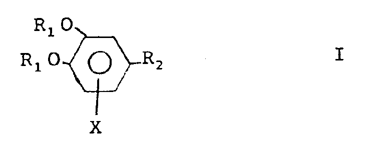 in which R₁ is hydrogen, X is nitro, cyano, trifluoromethyl, formyl or carboxyl and R₂ is a) halogen, substituted alkyl of up to 7 carbon atoms, alkoxy of up to 7 carbon atoms, an aryl or a heterocyclic ring, a nitro, cyano, formyl or carboxyl group or b) a group selected from 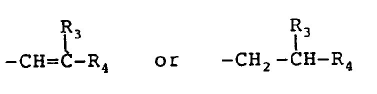 in which R₃ is hydrogen, alkyl of up to 7 carbon atoms, cyano, carboxyl, cycloalkyl carbonyl or acyl of up to 7 carbon atoms and R₄ is hydrogen, cyano, carboxyl, formyl, alkoxy of up to 7 carbon atoms, alkoxycarbonyl of up to 7 carbon atoms in the alkoxy, alkoxycarbonyl alkyl of up to 7 carbon atoms in the alkoxy and in the alkyl, carboxyalkenyl of up to 7 carbon atoms in the alkenyl, carboxycarbonyl, nitro, acyl of up to 7 carbon atoms, hydroxyalkyl of up to 7 carbon atoms, carboxyalkyl of up to 7 carbon atoms in the alkyl, or one of the following optionally substituted groups; carbamoyl, aryl, heterocyclic ring, cycloalkyl, cycloalkyl carbonyl, aroyl, or COZ, in which Z is an optionally substituted heterocyclic ring or c) 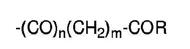 in which n is 0-1, m is 0-7 and R is alkyl of up to 7 carbon atoms, hydroxy, carboxyalkyl of up to 7 carbon atoms in the alkyl, optionally substituted alkenyl of up to 7 carbon atoms, optionally substituted heterocyclic ring or aryl, alkoxy of up to 7 carbon atoms or optionally substituted amino 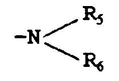 in which R₅ and R₆ independently comprise hydrogen or one of the following optionally substituted groups; alkyl of up to 7 carbon atoms, alkenyl of up to 7 carbon atoms, alkynyl of up to 7 carbon atoms, cycloalkyl, aralkyl or, taken together with nitrogen atom to which they are attached, form an optionally substituted heterocyclic ring; or a physiologically acceptable salt or ester thereof in the manufacture of a medicament for use in the prevention or treatment of tissue damage in a condition which is rheumatoid arthritis, a rejection reaction in an organ transplant, ischemia or ageing, the tissue damage being induced by lipid peroxidation.

2. Use according to claim 1, in which in the compound of formula I, X is nitro or cyano in the 5-position.

3. Use according to claim 1 or 2, in which in the compound of formula I, R₂ is 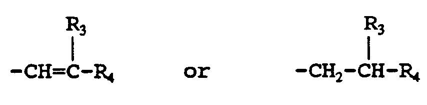 in which R₃ is carboxyl, cycloalkyl carbonyl or acyl of up to 7 carbon atoms and R₄ is carboxyl, acyl of up to 7 carbon atoms, formyl, alkoxycarbonyl of up to 7 carbon atoms, carboxycarbonyl, one of optionally substituted carbamoyl, cycloalkylcarbonyl, aroyl or COZ, in which Z is an optionally substituted heterocyclic ring.

4. Use according to claim 3, in which in the compound of formula I, at least one of R₃ and R₄ is acyl of up to 7 carbon atoms.

5. Use according to claim 3 or 4, in which in the compound of formula I, R₂ is substituted alkyl of up to 7 carbon atoms.

6. Use according to claim 1 or 2, in which R₂ is 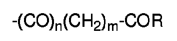 in which n is 0, m is 0 and R is optionally substituted amino or n is 0, m is 1 to 4 and R is alkyl up to 7 carbon atoms, hydroxy, carboxyalkyl of up to 7 carbon atoms, optionally substituted alkenyl of up to 7 carbon atoms optionally substituted heterocyclic ring or aryl, alkoxy of up to 7 carbon atoms or optionally substituted amino.

7. Use according to claim 1 or 2, in which R₂ is nitro or cyano.

8. Use according to claim 1, in which the compound of formula I is selected from the group comprising
N-(1-adamantyl)-3,4-dihydroxy-5-cyanobenzamide,
3,4-dihydroxy-5-nitrophenylmethanethiol,
4-[(3,4-dihydroxy-5-cyanophenyl)methylene]-3,5-heptanedione,
5-(3,4-dihydroxy-5-nitrophenyl)-2-methyl-4-phenyl-4-penten-3-one,
3,5-dicyanocatechol,
3,5-dinitrocatechol,
4-[(3,4-dihydroxy-5-cyanophenyl)methylene]-3,5-pentanedione,
1-(3,4-Di hydroxy-5-nitrophenyl)-2-methylene-1-propanone,
N, N-diethyl-2-cyano-3-(3,4-dihydroxy-5-nitrophenyl)acrylamide,
3,4-dihydroxy-2'-fluoro-5-nitrobenzophenone and
3,4-dihydroxy-4'-methyl-5-nitrobenzophenone.

9. Use according to claim 1, in which the compound of formula I is selected from the group comprising
3-nitro-5-[2-(4-pyridyl)vinyl]catechol,
3-nitro-5-[2-(4-quinolyl)vinyl]catechol,
3,5-dicyanocatechol,
N-(3-hydroxypropyl)-3,4-dihydroxy-5-nitrobenzamide,
neopentyl 2-cyano-3-(3,4-dihydroxy-5-nitrophenyl)acrylate and N-(3-hydroxypropyl)-2-cyano-3-(3,4-dihydroxy-
5-nitrophenyl)acrylamide
5-(3,4-dihydroxy-6-nitrophenyl)pentanoic acid,
N-(1-adamantyl)-3,4-dihydroxy-6-nitrobenzamide,
N-(4-morpholine ethyl)-3,4-dihydroxy-6-nitrobenzamidehydromesylate,
1-(3,4-dihydroxy-6-nitrobenzoyl)-4-cyclohexylcarbonyl-piperidine and
1-(3,4-dihydroxy-6-nitrobenzoyl)-4-(1-piperidyl)piperidine hydromesylate.

10. Use according to claim 4, in which the compound is 3-(3,4-dihydroxy-5-nitrobenzyl)-2,4-pentanedione.

11. Use according to claim 4, in which the compound is 3-(3,4-dihydroxy-5-nitrophenyl)methylene-2,4-pentanedione.

12. A catechol derivative which is a compound according to formula I 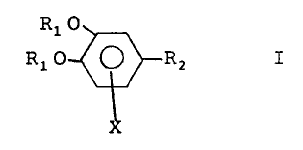 in which R₁ and X are as defined in claim 1, and R₂ is 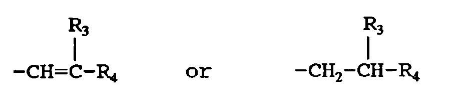 in which R₃ is acyl of up to 7 carbon atoms or cyclopropyl carbonyl and R₄ is optionally substituted aryl or cyclopropyl carbonyl or a physiologically acceptable salt or ester thereof.

13. 5-(2,4-Dihydroxy-5-nitrophenyl)-2-methyl-4-phenyl-4-perrten-3-one or a physiologically acceptable salt or ester thereof.

14. 3-(3,4-Dihydroxy-5-nitrobenzyl)-2,4-pentanedione or a physiologically acceptable salt or ester thereof.

15. 4-(3,4-Dihydroxy-5-nitrobenzyl)-3,5-heptanedione or a physiologically acceptable salt or ester thereof.

16. 1,3-Dicyclopropyl-2-[(3,4-dihydroxy-5-nitrophenyl)methylene]-1,3-propanedione or a physiologically acceptable salt or ester thereof.

17. 2-(3,4-Dihydroxy-5-nitrobenzyl)-1-cyclopropyl-1,3-butanedione or a physiologically acceptable salt or ester thereof.

18. A pharmaceutical composition comprising a compound according to claim 12 and a pharmaceutically acceptable carrier or diluent.

19. A compound as claimed in any one of claims 12 to 17 for use in a method of medical treatment by surgery, therapy or diagnosis.

## Patentansprüche

1. Verwendung eines Katechinderivats, das eine Verbindung darstellt mit der Formel (I) 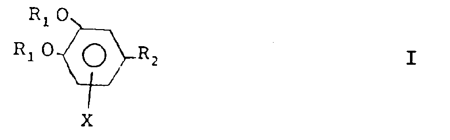 worin R₁ = Wasserstoff, X = Nitro, Cyano, Trifluormethyl, Formyl oder Carboxyl ist und R₂ = a) Halogen, substituiertes Alkyl mit bis zu 7 Kohlenstoffatomen, Alkoxy mit bis zu 7 Kohlenstoffatomen, ein Aryl oder ein heterozyklischer Ring, eine Nitro-, Cyano-, Formyl- oder Carboxylgruppe sind oder b) eine Gruppe, ausgewählt aus: 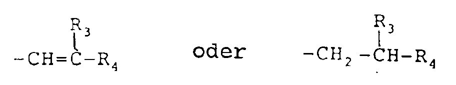 bedeuten, worin R₃ = Wasserstoff, Alkyl mit bis zu 7 Kohlenstoffatomen, Cyano, Carboxyl, Cycloalkyl-carbonyl oder Acyl mit bis zu 7 Kohlenstoffatomen ist und R₄ = Wasserstoff, Cyano, Carboxyl, Formyl, Alkoxy mit bis zu 7 Kohlenstoffatomen, Alkoxycarbonyl mit bis zu 7 Kohlenstoffatomen in der Alkoxyeinheit, Alkoxycarbonylalkyl mit bis zu 7 Kohlenstoffatomen in der Alkoxy- und in der Alkyleinheit, Carboxyalkenyl mit bis zu 7 Kohlenstoffatomen in der Alkenyleinheit, Carboxycarbonyl, Nitro, Acyl mit bis zu 7 Kohlenstoffatomen, Hydroxyalkyl mit bis zu 7 Kohlenstoffatomen, Carboxyalkyl mit bis zu 7 Kohlenstoffatomen in der Alkyleinheit, oder eine der folgenden, gegebenenfalls substitierten Gruppen bedeutet: Carbamoyl, Aryl, heterozyklischer Ring, Cycloalkyl, Cycloalkylcarbonyl, Aroyl oder COZ, worin Z ein gegebenenfalls substitutierter Ring ist oder c) 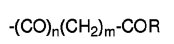 worin n = 0-1, m = 0-7 und R = Alkyl mit bis zu 7 Kohlenstoffatomen, Hydroxy, Carboxyalkyl mit bis zu 7 Kohlenstoffatomen in der Alkyleinheit, gegebenenfalls substituiertes Alkenyl mit bis zu 7 Kohlenstoffatomen, ein gegebenenfalls substituierter heterozyklischer Ring oder Aryl, Alkoxy mit bis zu 7 Kohlenstoffatomen oder gegebenenfalls substituiertes Amino 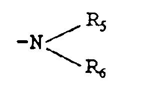 bedeutet, worin R₅ und R₆ unabhängig voneinander umfassen: Wasserstoff oder eine der folgenden, gegebenenfalls substituierten Gruppen: Alkyl mit bis zu 7 Kohlenstoffatomen, Alkenyl mit bis zu 7 Kohlenstoffatomen, Alkinyl mit bis zu 7 Kohlenstoffatomen, Cycloalkyl, Aralkyl oder - zusammen mit dem Stickstoffatomen, an das sie gebunden sind - einen gegebenenfalls substituierten heterozyklischen Ring bilden; oder ein physiologisch verwendbares Salz oder Ester davon, bei der Herstellung eines Medikaments zur Verwendung bei der Prävention oder Behandlung von Gewebeschäden bei Vorliegen von rheumatischer Arthritis, einer Abstoßungsreaktion bei Organtransplantation, Ischämie oder Altern, wobei der Gewebeschaden durch Lipidperoxidation induziert ist.

2. Verwendung nach Anspruch 1, wobei in der Verbindung der Formel X = Nitro oder Cyano in der 5-Stellung bedeutet.

3. Verwendung nach Anspruch 1 oder 2, wobei in der Verbindung der Formel I R₂ 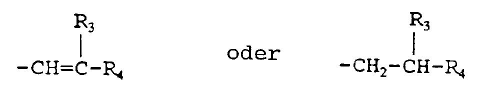 bedeutet, worin R₃ = Carboxyl, Cycloalkylcarbonyl oder Acyl mit bis zu 7 Kohlenstoffatomen ist und R₄ = Carboxyl, Acyl mit bis zu 7 Kohlenstoffatomen, Formyl, Alkoxycarbonyl mit bis zu 7 Kohlenstoffatomen, Carboxycarbonyl, eine der gegebenenfalls substituierten Gruppen: Carbamoyl, Cycloalkylcarbonyl, Aroyl oder COZ ist, wobei Z ein gegebenenfalls substituierter heterozyklischer Ring ist.

4. Verwendung nach Anspruch 3, wobei in der Verbindung der Formel wenigstens eine der Gruppen R₃ und R₄ Acyl mit bis zu 7 Kohlenstoffatomen darstellt.

5. Verwendung nach Anspruch 3 oder 4, wobei in der Verbindung der Formel I R₂ ein substituiertes Alkyl mit bis zu 7 Kohlenstoffatomen ist.

6. Verwendung nach Anspruch 1 oder 2, wobei R₂ 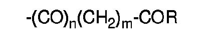 ist, worin n = 0, m = 0 und R ein gegebenenfalls substituiertes Amino ist, oder n = 0, m = 1 bis 4 und R = Alkyl mit bis zu 7 Kohlenstoffatomen, Hydroxy, Carboxyalkyl mit bis zu 7 Kohlenstoffatomen, gegebenenfalls substituiertes Alkenyl mit bis zu 7 Kohlenstoffatomen, ein gegebenenfalls substituierter heterozyklischer Ring oder Aryl, Alkoxy mit bis zu 7 Kohlenstoffatomen oder ein gegebenenfalls substituiertes Amino bedeutet.

7. Verwendung nach Anspruch 1 oder 2, wobei R₂ = Nitro oder Cyano bedeutet.

8. Verwendung nach Anspruch 1, wobei die Verbindung der Formel ausgewählt ist aus der Gruppe, welche umfaßt:
N-(1-Adamantyl)-3, 4-dihydroxy-5-cyanobenzamid,
3,4-Dihydroxy-5-nitrophenylmethanethiol,
4-[(3,4-Dihydroxy-5-cyanophenyl)methylen]-3,5-heptandion,
5-(3,4-Dihydroxy-5-nitrophenyl)-2-methyl-4-phenyl-4-penten-3-on,
3,5-Dicyanobrenzcatechin,
3,5-Dinitrobrenzcatechin,
4-[(3,4-Dihydroxy-5-cyanophenyl)methylen]-3,5-pentandion,
1-(3,4-Dihydroxy-5-nitrophenyl)-2-methylen-1-propanon,
N, N-Diethyl-2-cyano-3-(3,4-dihydroxy-5-nitrophenyl)-acrylamid,
3,4-Dihydroxy-2'-fluor-5-nitrobenzophenon und
3,4-Dihydroxy-4'-methyl-5-nitrobenzophenon.

9. Verwendung nach Anspruch 1, wobei die Verbindung der Formel 1 ausgewählt ist aus der Gruppe, die umfaßt:
3-Nitro-5-[2-[4-pyridyl)vinyl]brenzcatechin,
3-Nitro-5-[2-(4-chinolyl)vinyl]brenzcatechin,
3,4-Dihydroxy-5-nitrozimtsäure
3,4-Dihydroxy-5-nitro-ω,ω-dicyanostyrol
4-(3,4-Dihydroxy-5-nitrophenyl)-3-methylbut-3-en-2-on,
3-(3,4-Dihydroxy-5-nitrophenyl)methylen-2,4-pentandion,
3-(3,4-Dihydroxy-5-nitrophenyl)-1-phenylprop-2-en-1-on,
3-(3,4-Dihydroxy-5-nitrophenyl)-1-(4-methoxyphenyl)-prop-2-en-1-on,
3-(3,4-Dihydroxy-5-nitrophenyl)-1-(3,4-dimethoxyphenyl)-prop-2-en-1-on,
3-(3,4-Dihydroxy-5-nitrophenyl)-1-(3, 4,5-trimethoxyphenyl)-prop-2-en-1-on,
3-(3,4-Dihydroxy-5-nitrophenyl)-1-(2-hydroxyphenyl)prop-2-en-1-on,
3-(3,4-Diacetoxy-5-nitrophenyl)-1-phenylprop-2-en-1-on,
3-(3,4-Dibenzoyloxy-5-nitrophenyl)-1-phenylprop-2-en-1-on,
3-(3-Pivaloyloxy-4-hydroxy-5-nitrophenyl)-1-phenyl-prop-2-en-1-on,
4-(3,4-Dihydroxy-5-nitrophenyl)-3-methylbut-3-en-2-ol,
3',4'-Dihydroxy-5-nitroacetophenon,
5-(3,4-Dihydroxy-5-nitrophenyl)pentansäure,
1-Benzyl-4-[5-(3,4-dihydroxy-5-nitrophenyl)penanoyl]-piperazin-hydrochlorid,
N-Isopropyl-5-(3,4-dihydoxy-5-nitrophenyl)pentansäureamid,
N-Methyl-N-propargyl-5-(3,4-dihydroxy-5-nitrophenyl)-pentansäureamid,
N-(1-Adamantyl)-5-(3,4-dihydroxy-5-nitrophenyl)-pentansäureamid,
3-(3,4-Dihydroxy-5-nitrophenyl)-1-(4-methylphenyl)-prop-2-en-1-on,
N-(1-Adamantyl)-3,4-dihydroxy-5-nitrobenzamid,
4-Cyclohexylcarbonyl-1-(3,4-dihydroxy-5-nitrobenzoyl)-piperidin,
N-Benzyl-3,4-dihydroxy-5-nitrobenzamid,
N-(1-Adamantyl)-3,4-dihydroxy-5-chlorbenzamid,
N-(1-Adamantyl)-3,4-dihydroxy-5-cyanobenzamid,
1-Butyl-3,4-dihydroxy-5-cyanobenzoat,
3-(3-Ethoxycarbonylmethylcarbamoyloxy-4-hydroxy-5-nitrophenyl)-1-phenylprop-2-en-1-on,
3-(3,4-Dihydroxy-5-nitrophenyl)-1-(2-carboxyphenyl)-prop-2-en-1-on,
3-(3,4-Dihydroxy-5-nitrophenyl)-1-(4-nitrophenyl)-prop-2-en-1-on,
Ethyl-2-cyano-3-(3,4-dihydroxy-5-nitrophenyl)acrylat,
Methyl-3-(3,4-dihydroxy-5-nitrophenyl)methylen-4-ketopentanoat,
3,4-Dihydroxy-5-nitrobenzylmalonitril, Ethyl-3,4-dihydroxy-5-nitrobenzylcyanoacetat,
3-(3,4-Dihydroxy-5-trifluormethylphenyl)-prop-2-en-1-on,
3,4-Dihydroxy-5-cyanobenzaldehyd,
3,4-Dihydroxy-5-trifluormethylbenzaldehyd,
2-Cyano-3-(3,4-dihydroxy-5-nitrophenyl)acrylamid,
N, N-Dimethyl-2-cyano-3-(3,4-dihydroxy-5-nitrophenyl)-acrylamid,
N, N-Diethyl-2-cyano-3-(3,4-dihydroxy-5-nitrophenyl)acrylamid,
N-Isopropyl-2-cyano-3-(3,4-dihydroxy-5-nitrophenyl)-acrylamid,
N'-Methyl-N"-[2-cyano-3-(3,4-dihydroxy-5-nitrophenyl)-acryl]piperazin,
3-(3,4-Dihydroxy-5-tr'rfluormethylphenyl)methylen-2,4-pentandion,
3,4-Dihydroxy-5-nitrobenzylalkohol,
3,4-Dihydroxy-5-nitrobenzyl-2-methoxyethylether,
3,4-Dihydroxy-5-nitrobenzylthioessigsäure,
2-(3,4-Dihydroxy-5-nitrobenzyl)pyrrol
2-Cyano-3-(3,4-dihydroxy-5-nitrophenyl)propanol,
3,5-Dinitrobrenzcatechin,
3,4-Dihydroxy-5-nitrobenzaldehyd,
3,4-Dihydroxy-5-nitrobenzonitril,
4-Chlor-6-nitrocatechin,
4,5-Dihydroxyisophthalaldehyd,
3,4-Dihydroxy-5-cyanobenzoesäure,
3,5-Dicyanobrenzcatechin,
N-(3-Hydroxypropyl)-3,4-dihyroxy-5-nitrobenzamid,
Neopentyl-2-cyano-3-(3,4-dihydroxy-5-nitrophenyl)acrylat und
N-(3-Hydroxypropyl)-2-cyano-3-(3,4-dihydroxy-5-nitrophenyl)acrylamid,
5-[3,4-Dihydroxy-6-nitrophenyl)pentansäure,
N-(1 -Adamantyl)-3,4-dihydroxy-6-nitrobenzamid,
N-(4-Morpholinethyl)-3,4-dihydroxy-6-nitrobenzamid-hydromesylat,
1-(3,4-Dihydroxy-6-nitrobenzoyl)-4-cyclohexylcarbonyl-piperidin und
1-(3,4-Dihydroxy-6-nitrobenzoyl)-4-(1-piperidyl)piperidinhydromesylat.

10. Verwendung nach Anspruch 4, wobei die Verbindung 3-(3,4-Dihydroxy-5-nitrobenzyl)-2,4-pentandion ist.

11. Verwendung nach Anspruch 4, wobei die Verbindung 3-(3,4-Dihydroxy-5-nitrobenzyl)methylen-2,4-pentandion ist.

12. Katechinderivat, das eine Verbindung mit der Formel 1 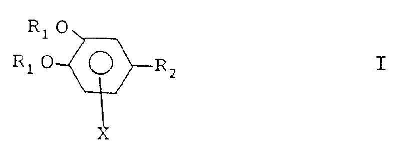 darstellt, worin R₁ und X die in Anspruch 1 definierten Bedeutungen haben, und R₂ 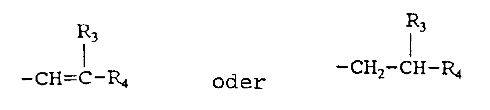 ist, worin R₃ = Acyl mit bis zu 7 Kohlenstoffatomen oder Cyclopropyl-carbonyl ist und R₄ ein gegebenenfalls substituiertes Aryl oder Cyclopropyl-carbonyl ist oder ein physiologisch verwendbares Salz oder Ester von diesen.

13. 5-(2,4-Dihydroxy-5-nitrophenyl)-2-methyl-4-phenyl-4-penten-3-on oder ein physiologisch verwendbares Salz oder Ester davon.

14. 3-(3,4-Dihydroxy-5-nitrobenzyl)2,4-pentandion oder ein physiologisch verwendbares Salz oder Ester davon.

15. 4-(3,4-Dihydroxy-5-nitrobenzyl)-3,5-heptandion oder ein physiologisch verwendbares Salz oder Ester davon.

16. 1,3-Dicyclopropyl-2-[(3,4-dihydroxy-5-nitrophenyl)methylen]-1,3-propandion oder ein physiologisch verwendbares Salz oder Ester davon.

17. 2-(3,4-Dihydroxy-5-nitrobenzyl)-1-cyclopropyl-1,3-butandion oder ein physiologisch verwendbares Salz oder Ester davon.

18. Pharmazeutische Zusammensetzung, die eine Verbindung nach Anspruch 12 und einen pharmazeutisch verwendbaren Träger oder Verdünnungsmittel umfaßt.

19. Verbindung nach einem der Ansprüche 12 bis 17 zur Verwendung bei einem Verfahren der medizinischen Behandlung in der Chirurgie, Therapie oder Diagnose.

## Revendications

1. Utilisation d'un dérivé du catéchol qui est un composé de formule (I): 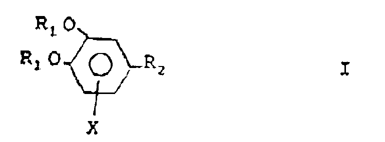 dans laquelle
- R₁ est un atome d'hydrogène, X est un groupe nitro, cyano, trifluorométhyle, formyle, ou carboxyle, et
- R₂ représente
(a) un halogène, un groupe alkyle substitué comportant jusqu'à 7 atomes de carbone, un groupe alcoxy comportant jusqu'à 7 atomes de carbone, un groupe alkyle, ou un hétérocycle, un groupe nitro, cyano, ou carboxyle, ou
(b) un groupe choisi parmi 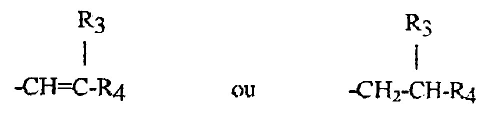 où R₃ est un atome d'hydrogène, un groupe alkyle comportant jusqu'à 7 atomes de carbone, un groupe cyano, carboxyle, cycloalkylcarbonyle ou acyle comportant jusqu'à 7 atomes de carbone, et R₄ est un atome d'hydrogène un groupe cyano, carboxyle, formyle, alcoxy comportant jusqu'à 7 atomes de carbone, alcoxycarbonyle comportant jusqu'à 7 atomes de carbone dans l'alcoxy, un groupe alcoxycarbonylalkyle comportant jusqu'à 7 atomes de carbone dans l'alcoxy et dans l'alkyle, un groupe carboxyalkényle comportant jusqu'à 7 atomes de carbone dans l'alkényle, un groupe carboxycarbonyle, nitro, acyle comportant jusqu'à 7 atomes de carbone, un groupe hydroxyalkyle comportant jusqu'à 7 atomes de carbone, un groupe carboxyalkyle comportant jusqu'à 7 atomes de carbone dans l'alkyle, ou un des groupes suivants éventuellement substitués : carbamoyle, aryle, hétérocyclique, cycloalkyle, cycloalkylcarbonyle, aroyle, ou COZ, dans lequel Z est un hétérocycle éventuellement substitué, ou
(c) un groupe 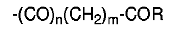 dans lequel n est égal à 0 ou à 1, m est compris entre 0 et 7, et R est un groupe alkyle comportant jusqu'à 7 atomes de carbone, un groupe hydroxy, un groupe carboxyalkyle comportant jusqu'à 7 atomes de carbone dans l'alkyle, un groupe alkényle éventuellement substitué, comportant jusqu'à 7 atomes de carbone, un groupe hétérocyclique ou aryle éventuellement substitué un groupe alcoxy comportant jusqu'à 7 atomes de carbone, ou un groupe amino éventuellement substitué, 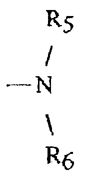 dans lequel R₅ et R₆ comprennent indépendamment, l'hydrogène ou l'un des groupes suivants, éventuellement substitué : alkyle comportant jusqu'à 7 atomes de carbone, alkényle comportant jusqu'à 7 atomes de carbone, alkynyle comportant jusqu'à 7 atomes de carbone, cycloalkyle, aralkyle, ou bien pris ensemble, avec l'atome d'azote auquel ils sont attachés, forment un hétérocycle éventuellement substitué ; ou d'un sel ou d'un ester de celui-ci, physiologiquement acceptable, dans la fabrication d'un médicament en vue de l'utilisation dans la prévention ou le traitement des dommages de tissu dans un état tel que l'arthrite rhumatoïde, une réaction de rejet d'un organe transplanté, l'ischémie, ou le vieillissement, le dommage de tissu étant induit par la peroxydation de la graisse.

2. Utilisation selon la revendication 1, pour laquelle, dans le composé de formule (I), X représente un groupe nitro ou cyano, en position 5.

3. Utilisation selon la revendication 1, ou la revendication 2, pour laquelle, dans le composé de formule (1), R₂ représente : 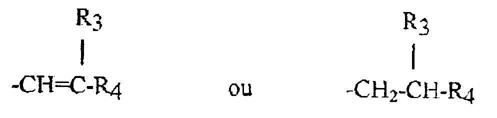 où R₃ est un groupe carboxyle, cycloalkylcarbonyle ou acyle comportant jusqu'à 7 atomes de carbone, et R₄ est un groupe carboxyle, acyle comportant jusqu'à 7 atomes de carbone, formyle, alcoxycarbonyle comportant jusqu'à 7 atomes de carbone, carboxycarbonyle, un des groupes carbamoyle, cycloalkylcarbonyle, aroyle, ou COZ, éventuellement substitué, dans lequel Z est un hétérocycle éventuellement substitué.

4. Utilisation selon la revendication 3, pour laquelle, dans le composé de formule (1), au moins l'un de R₃ ou de R₄ est un groupe acyle comportant jusqu'à 7 atomes de carbone.

5. Utilisation selon la revendication 3, ou la revendication 4, pour laquelle, dans le composé de formule (I), R₂ est un groupe alkyle substitué comportant jusqu'à 7 atomes de carbone.

6. Utilisation selon la revendication 1, ou la revendication 2, pour laquelle R₂ représente 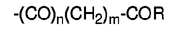 où n est égal à 0, m est égal à 0, et R est un groupe amino éventuellement substitué, ou bien n est égal à 0, m est compris entre 1 et 4 et R est un groupe alkyle comportant jusqu'à 7 atomes de carbone, un groupe hydroxy, un groupe carboxyalkyle comportant jusqu'à 7 atomes de carbone, un groupe alkényle éventuellement substitué, comportant jusqu'à 7 atomes de carbone, un groupe hétérocyclique ou aryle éventuellement substitué, un groupe alcoxy comportant jusqu'à 7 atomes de carbone, ou un groupe amino éventuellement substitué.

7. Utilisation selon la revendication 1, ou la revendication 2, pour laquelle R₂ est un groupe nitro ou cyano.

8. Utilisation selon la revendication 1, dans laquelle le composé de formule (I) est choisi au sein du groupe comprenant :
- le N-(1-adamantyl)-3,4-dihydroxy-5-cyanobenzamide,
- le 3,4-dihydroxy-5-nitrophénylméthanethiol,
- la 4-[(3,4-dihydroxy-5-cyanophényl)méthylène]-3,5-heptanedione,
- la 5-(3,4-dihydroxy-5-nitrophényl)-2-méthyl-4-phényl-4-pentèn-3-one,
le 3,5-dicyanocatéchol,
- le 3,5-dinitrocatéchol,
la 4-[(3,4-dihydroxy-5-cyanophényl)méthylène]-3,5-pentanedione,
- la 1-(3,4-dihydroxy-5-nitrophényl)-2-méthylène-1-propanone,
- le N,N-diéthyl-2-cyano-3-(3,4-dihydroxy-5-nitrophényl)-acrylamide,
- la 3,4-dihydroxy-2'-fluoro-5-nitrobenzophénone, et
- la 3,4-dihydroxy-4'-méthyl-5-nitrobenzophénone.

9. Utilisation selon la revendication 1, pour laquelle le composé de formule (I) est choisi au sein du groupe comprenant :
- le 3-nitro-5-[2-(4-pyridyl)vinyl]catéchol,
- le 3-nitro-5-[2-(4-quinoléyl)vinyl]catéchol,
- l'acide 3,4-dihydroxy-5-nitrocinnamique,
- le 3,4-dihydroxy-5-nitro-w,w-dicyanostyrène,
- la 4-(3,4-dihydroxy-5-nitrophény)-3-méthylbut-3-èn-2-one,
- la 3-(3,4-dihydroxy-5-nitrophényl)méthylène-2,4-pentanedione,
- la 3-(3,4-dihydroxy-5-nitrophényl)méthylène-2,4-pentanedione,
- la 3-(3,4-dihydroxy-5-nitrophényl)-1-(4-méthoxyphényl)prop-2-èn-1-one
- la 3-(3,4-dihydroxy-5-nitrophényl)-1-(3,4-diméthoxyphényl)prop-2-èn-1-one,
- la 3-(3,4-dihydroxy-5-nitrophényl)-1-(3,4,5-triméthoxyphényl)prop-2-èn-1-one,
- la 3-(3,4-dihydroxy-5-nitrophényl)-1-(2-hydroxyphényl)prop-2-èn-1-one,
- la 3-(3,4-diacétoxy-5-nitrophényl)-1-phénylprop-2-èn-1-one,
- la 3-(3,4-dibenzoyloxy-5-nitrophényl)-1-phénylprop-2-èn-1-one,
- la 3-(3-pivaloyloxy-4-hydroxy-5-nitrophényl)-1-phénylprop-2-èn-1-one,
- le 3-(3,4-dihydroxy-5-nitrophényl)-3-méthylbut-3-èn-2-ol,
- la 3',4'-dihydroxy-5'-nitroacétophénone,
- l'acide 5-(3,4-dihydroxy-5-nitrophényl)pentanoïque,
- le chlorhydrate de 1-benzyl-4-[5-(3,4-dihydroxy-5-nitrophényl)pentanoyl]pipérazine,
- l'amide N-isopropyl-5-(3,4-dihydroxy-5-nitrophényl)pentanoïque,
- l'amide N-méthyl-N-propargyl-5-(3,4-dihydroxy-5-nitrophényl)pentanoïque,
- l'amide N-(1-adamantyl)-5-(3,4-dihydroxy-5-nitrophényl)pentanoïque,
- la 3-(3,4-dihydroxy-5-nitrophényl)-1-(4-méthylphényl)prop-2-èn-1-one,
- le N-(1-adamantyl)-3,4-dihydroxy-5-nitrobenzamide,
- la 4-cyclohexylcarbonyl-1-(3,4-dihydroxy-5-nitrobenzyl)pipéridine,
- le N-benzyl-3,4-dihydroxy-5-nitrobenzamide,
- le N-(1-adamantyl)-3,4-dihydroxy-5-chlorobenzamide,
- le N-(1-adamantyl)-3,4-dihydroxy-5-cyanobenzamide,
- le 1-butyl-3,4-dihydroxy-5-cyanobenzoate,
- la 3-(3-éthoxycarbonylméthylcarbamoyloxy-4-hydroxy-5-nitrophényl)-1-phénylprop-2-èn-1-one, la 3-(3,4-d!hydroxy-5-nitrophényl)-1-(2-carboxyphényl)prop-2-èn-1-one,
- la 3-(3,4-dihydroxy-5-n!trophényl)-1-(4-nitrophényl)prop-2-èn-1-one,
- l'éthyle 2-cyano-(3,4-dihydroxy-5-nitrophényl)acrylate,
- le méthyle 3-(3,4-dihydroxy-5-nitrophényl)méthylène-4-cétopentanoate,
- le 3,4-dihydroxy-5-nitrobenzylmalonitrile,
- l'éthyle 3,4₋dihydroxy-5-nitrobenzylcyanoacétate,
- la 3-(3,4-dihydroxy-5-trifluorométhylphényl)prop-2-èn-1-one,
- le 3,4-dihydroxy-5-cyanobenzaldéhyde,
- le 3,4-dihydroxy-5-trifluorométhylbenzaldéhyde,
- le 2-cyano-3-(3,4-dihydroxy-5-nitrophényl)acrylamide,
- le N,N-diméthyl-2-cyano-3-(3,4-dihydroxy-5-nitrophényl)acrylamide,
- le N,N-diéthyl-2-cyano-3-(3,4-dihydroxy-5-nitrophényl)acrylamide,
- le N-isopropyl-2-cyano-3-(3,4-dihydroxy-5-nitrophényl)acrylamide,
- la N'-méthyl-N"-[2-cyano-3-(3,4-dihydroxy-5-nitrophényl)acryl]pipérazine,
- la 3-(3,4-dihydroxy-5-trifluorométhylphényl)]méthylène-2,4-pentadione,
- l'alcool 3,4-dihydroxy-5-nitrobenzylique.
- l'éther 3,4-dihydroxy-5-nitrobenzyl-2-méthoxyéthyle,
- l'acide 3,4-dihydroxy-5-nitrobenzylthioacétique,
- le 2-(3,4-dihydroxy-5-nitrobenzyl)pyrrole,
- le 2-cyano-3-(3,4-dihydroxy-5-nitrophényl)propanol,
- le 3,5-dinitrocatéchol,
- le 3,4-dihydroxy-5-nitrobenzaldéhyde,
- le 3,4-dihydroxy-5-nitrobenzonitrile,
- le 4-chloro-6-nitrocatéchol,
- le 4,5-dihydroxyisophtalaldéhyde,
- l'acide 3,4-dihydroxy-5-cyanobenzoïque,
- le 3,5-dicyanocatéchol,
- le N-(3-hydroxypropyl)-3,4-dihydroxy-5-nitrobenzamide,
- le néopentyle 2-cyano-3-(3,4-dihydroxy-5-nitrophényl)acrylate,
- le N-(3-hydroxypropyl)-2-cyano-3-(3,4-dihydroxy-5-nitrophényl)acrylamide,
- l'acide 5-(3,4-dihydroxy-6-nitrophényl)pentanoïque,
- le N-(1-adamantyl)-3,4-dihydroxy-6-nitrobenzamide,
- le N-(4-morpholine éthyl)-3,4-dihydroxy-6-nitrobenzamide hydromésylate,
- la 1-(3,4-dihydroxy-6-nitrobenzoyl)-4-cyclohexylcarbonylpipéridine, et
- le 1-(3,4-dihydroxy-6-nitrobenzoyl)-4-(1-pipéridyl)pipéridine hydromésylate.

10. Utilisation selon la revendication 4, pour laquelle le composé est la 3-(3,4-dihydroxy-5-nitrobenzyl)-2,4-pentanedione.

11. Utilisation selon la revendication 4, pour laquelle le composé est la 3-(3,4-dihydroxy-5-nitrophényl)méthylène-2,4-pentanedione.

12. Dérivé du catéchol, qui est un composé de formule (I) 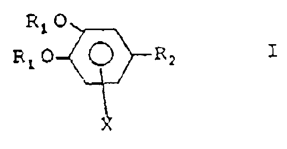 dans laquelle R₁ et X sont tels que définis dans la revendication 1, et R₂ représente 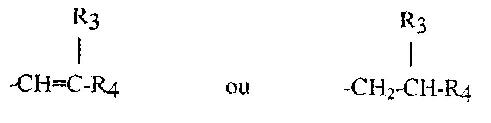 où R₃ est un groupe acyle comportant jusqu'à 7 atomes de carbone, ou un groupe cyclopropylcarbonyle, et R₄ est un groupe aryle ou cyclopropylcarbonyle éventuellement substitué,
ou un sel ou ester de celui-ci, physiologiquement acceptable.

13. La 5-(2,4-dihydroxy-5-nitrophényl)-2-méthyl-4-phényl-4-pentèn-3-one ou un sel ou ester de celle-ci, physiologiquement acceptable.

14. La 3-(3,4-dihydroxy-5-nitrobenzyl)-2,4-pentanedione ou un sel ou ester de celle-ci, physiologiquement acceptable.

15. La 3-(3,4-dihydroxy-5-nitrobenzyl)-3,5-heptanedione ou un sel ou ester de celle-ci, physiologiquement acceptable.

16. La 1,3-dicyclopropyl-2-[(3,4-dihydroxy-5-nitrophényl)méthylène]-1,3-propanedione ou un sel ou ester de celle-ci, physiologiquement acceptable.

17. La 2-(3,4-dihydroxy-5-nitrobenzyl)-1-cyclopropyl-1,3-butanedione ou un sel ou ester de celle-ci, physiologiquement acceptable.

18. Composition pharmaceutique contenant un composé selon la revendication 12, et un véhicule ou diluant pharma- ceutiquement acceptable.

19. Composé selon l'une quelconque des revendications 12 à 17, en vue de l'utilisation dans une méthode de traitement médical par chirurgie, thérapie ou diagnostic.
